# EUROPÄISCHE PATENTSCHRIFT

(11) **EP 1 957 970 B1**
(45) Veröffentlichungstag und Bekanntmachung des Hinweises auf die Patenterteilung: **13.01.2010**
(21) Anmeldenummer: 06819832.4
(22) Anmeldetag: 29.11.2006
(51) Int. Cl.: G01N 33/34, G01N 27/60

(54) **VORRICHTUNG FÜR DIE STRÖMUNGSPOTENTIALMESSUNG VON FASERN UND PARTIKELN IN SUSPENSIONEN**
DEVICE FOR MEASURING THE STREAMING POTENTIAL OF FIBERS AND PARTICLES IN SUSPENSIONS
DISPOSITIF DE MESURE DU POTENTIEL D'ECOULEMENT DE FIBRES ET DE PARTICULES DANS DES SUSPENSIONS

(30) Priorität: 10.12.2005 DE 202005019336 U; 12.01.2006 DE 202006000403 U; 22.02.2006 DE 102006008569
(43) Veröffentlichungstag der Anmeldung: 20.08.2008
(73) Patentinhaber: emtec Electronic GmbH, 04347 Leipzig (DE); AFG Analytic GmbH, 04347 Leipzig (DE)
(72) Erfinder: GRÜNER, Giselher, 04129 Leipzig (DE)
(74) Vertreter: Schneider, Henry
(86) Internationale Anmeldenummer: PCT/EP2006/069050
(87) Internationale Veröffentlichungsnummer: WO 2007/065825

(56) Entgegenhaltungen:
- DE-A1- 3 130 529
- DE-A1- 10 200 654
- DE-U1- 20 209 563
- US-A- 3 368 392
- US-A- 5 314 581

## Beschreibung

Die Erfindung betrifft eine Vorrichtung für die Strömungspotentialmessung von Fasern und Partikeln in Suspensionen, insbesondere eine Vorrichtung zur Bestimmung des Zetapotentials der Teilchen von faser- bzw. partikelhaltigen wässrigen Suspensionen durch Messung des Strömungspotentials und einer daraus erfolgenden Berechnung des Zetapotentials mittels einer empirischen Formel.

Die Charakterisierung von Oberflächen und Grenzflächen und der dort stattfindenden Wechselwirkungen ist bedeutsam für vielfältige Fragestellungen zu chemischen, biotechnologischen und medizintechnischen Vorgängen. So auch, um insbesondere zur Beurteilung wässriger Suspensionen, die Feststoffe, Emulgatoren, Fasern und sonstige Partikel enthalten. Sehr bedeutsam ist die Charakterisierung von Oberflächen und Grenzflächen und den an diesen stattfindenden Wechselwirkungen für die Papierherstellung. Elektrische Effekte an Fest-Flüssig-Phasengrenzen, die elektrischen Doppelschichten und das damit verbundene Zetapotential des Feststoffes sind charakteristisch für das jeweilige Material und dessen aktuelle Umgebung. Das elektrische Potential der Feststoffoberfläche beeinflusst die Adsorption und Haftung von Substanzen aus dem entsprechenden Milieu. Aussagen über die Größe und das Vorzeichen der Oberflächenladung können durch Messung des so genannten Zetapotentials erhalten werden, welches die Galvani-Spannung an der diffusen elektrochemischen Doppelschicht an der Phasengrenze zwischen der Oberfläche eines Feststoffes und einer Flüssigkeit beschreibt.

In der Papierindustrie ist das Zetapotential von Fasern in der Faserstoffsuspension ein wichtiger Parameter, um eine optimale Prozessführung zu gewährleisten. Gleiches gilt für Textilfasern in der Textilindustrie und für viele Arten von Teilchen in Verarbeitungsprozessen. Zur Ermittlung dieses Potentials gibt es verschiedene Verfahren, wie z. B.:
- Messung der Wanderungsgeschwindigkeit der Partikel in der Suspension in einem elektrischen Feld und daraus Berechnung des Zetapotentials,
- Messung des Strömungspotentials der Fasern oder Teilchen in der Suspension, Berechnung des Zetapotentials aus diesem gemessenen Strömungspotential mit Hilfe einer empirischen Formel.

Aus der EP 0 462 703 B1 ist ein Gerät zur Messung einer elektrischen Eigenschaft, einer Faserdispersion, bekannt. Diese Vorrichtung zur Messung einer druckabhängigen Charakteristik einer Dispersion aus Festmaterial in einem Fluid besteht aus einem Mittel zur Durchführung wenigstens eines Teiles des Fluids von einer ersten Kammer durch ein Sieb in eine zweite Kammer zur Bildung eines Kissens aus Festmaterial auf dem Sieb und Mitteln zur Messung der Charakteristik. Diese Vorrichtung weist eine Druckregeleinrichtung mit wenigstens einem Differentialdruckregler auf, wobei der Differentialdruckregler so angeordnet ist, dass ein Drucksignal entsprechend einem vorbestimmten Druckwert vorzugeben ist, und weist ferner Mittel auf, um Luft aus einer zweiten Kammer mit einer definierten Geschwindigkeit zu ziehen.

Eine Zetapotential-Messzelle wird in der DE 43 45 152 A1 beschrieben. Die Zetapotential-Messzelle zur Ermittlung des Zetapotentials an äußeren und/oder quellenden Oberflächen strömungsstabiler Materialien enthält einen Körper, der mit mindestens zwei in einem Winkel von 90° zueinander stehenden und sich schneidenden Durchbohrungen versehen ist. In der einen Durchbohrung sind von jeder Öffnung der Durchbohrung im Körper her ein dreh- und verschiebbarer Stempel, der gegen den Körper abgedichtet ist, bis auf Messspaltweite eingeschoben. Die Stirnflächen der Stempel im Körper sind eben und parallel zueinander und zwischen ihnen befindet sich ein Messspalt. Die andere Durchbohrung ist als Zu- und Abführungskanal einer elektrolytischen Flüssigkeit ausgebildet und es befindet sich im Zu- und im Abführungskanal je eine Elektrode. Die Oberfläche des Zu- und Abführungskanals und der Stempel im Körper verhindern eine direkte elektrische Verbindung zwischen den beiden Elektroden.

Die WO 97/36173 A1 beschreibt eine Vorrichtung zur Bestimmung der Ladungsdichte von gelösten, kolloidal gelösten oder ungelösten, organischen oder anorganischen Substanzen in einer Probeflüssigkeit mittel Titration, umfassend ein mit mindestens zwei Elektroden versehenes Messgefäß zur Aufnahme der Probeflüssigkeit, in welchem ein Kolben in der Probeflüssigkeit um eine Betriebsstellung herum motorisch bewegbar ist, **dadurch gekennzeichnet, dass** Abstreifmittel zur mechanischen Reinigung von Kolben und Gefäß vorgesehen sind.

Eine Gerät zur elektrokinetischen Analyse mit minimalem Flüssigkeitsvolumen wird in der DE 202 09 563 U1 beschrieben. Das Gerät ist **dadurch gekennzeichnet, dass** eine oszillierende Flüssigkeitsströmung derart erzeugt wird, dass mit geringen Proben- und Flüssigkeitsvolumina gearbeitet werden kann, wobei bei faserförmigem, pulverförmigem oder granuliertem Probematerial das Verhältnis des Flüssigkeitsvolumens zum Packvolumen der festen Probe nicht größer als 10:1, und bei planaren Proben das Verhältnis des Flüssigkeitsvolumens zur festen Oberfläche nicht größer als 0,5 cm³/cm² sein muss.

Weitere Vorrichtungen zur Messung des Strömungspotentials von faser- bzw. partikelhaltigen wässrigen Suspensionen sind mit vielfältigsten Funktionsprinzipien bekannt. Bei diesen Vorrichtungen wird beispielsweise in einer nach unten offenen, mit einem Ansaugrohr versehenen Messzelle, die an der Oberseite einseitig mit einem Sieb abgeschlossen ist, ein Faserpfropfen bzw. ein Partikelpfropfen erzeugt. Das geschieht durch Auffüllen der Messzelle mittels Ansaugen der Suspension aus z. B. einem Becherglas, indem an die Messzelle auf einer oben befindlichen Siebseite ein definiertes Vakuum angelegt wird. Dieses Vakuum wird von einer extern angeordneten Vakuumpumpe erzeugt, die mit einem mit der Messzelle verbundenen Gefäß, das sich oberhalb der Messzelle befindet und dessen Volumen so bemessen ist, dass es das gesamte Filtrat/Elektrolyt der Suspension aufnehmen kann, verbunden ist.

Über das an der unteren Seite der Messzelle befindliche, vorzugsweise senkrechte Ansaugrohr wird die Suspension angesaugt und der für die Messung erforderliche Pfropfen gebildet, indem das Sieb nur Wasser bzw. Elektrolyt, jedoch praktisch keine Fasern oder Partikel passieren lässt. Durch den Unterdruck wird gleichzeitig der gebildete Pfropfen in der für eine korrekte Messung erforderlichen Weise verdichtet. Durch das auf der Siebseite permanent anliegende Vakuum wird sichergestellt, dass der Pfropfen in seiner Position bleibt. Das auf Grund des Unterdruckes durch den Pfropfen angesaugte Filtrat sammelt sich in dem Vakuumbehälter.

Nach Bildung des Pfropfens oder auch während dessen Bildung wird durch eine periodische Veränderung des Unterdruckes eine periodisch sich ändernde Strömung des Wassers der Suspension aus dem Becherglas durch den Pfropfen erzeugt. Diese Strömung erzeugt durch Deformation von Ladungswolken, die um die Fasern bzw. Teilchen herum angeordnet sind, eine periodisch sich ändernde Spannung. Die Frequenz dieser periodischen Änderung liegt etwa im Bereich von 0,5 Hz bis 10 Hz. Das so genannte Strömungspotential wird mittels zweier Elektroden, die sich an den beiden Enden der Messzelle befinden, gemessen. Die Elektroden bestehen beispielsweise aus Edelstahl, Platin, Silber oder Gold.

Aus dem sich periodisch ändernden Strömungspotential und der mittels eines Drucksensors gemessenen ebenfalls periodisch sich ändernden Druckdifferenz zum Umgebungsdruck sowie weiteren Größen wird das Zetapotential berechnet. Der Vorteil der periodischen Spannungsänderung liegt darin, dass Offsetgleichspannungen, die z. B. durch Verschmutzungen und Beläge auf den Elektroden entstehen, herausgefiltert werden können. Das periodisch sich ändernde Vakuum, das die periodische Strömung durch den Pfropfen erzeugt, wird durch eine leistungsstarke Vakuumpumpe erzeugt, die über zwei Druckreduzierventile und zwei nachgeschaltete Ventile an ein Vakuumgefäß angeschlossen ist und die permanent während er gesamten Messung aktiv ist. Durch wechselweises Umschalten der Ventile ändert sich das Vakuum im Gefäß jeweils entsprechend dem über zwei Druckreduzierer eingestellten Unterdruck. Das Gefäß nimmt gleichzeitig das gemeinsam mit der Suspension aus dem Becherglas angesaugte, durch den Pfropfen strömende und durch ihn gefilterte Wasser bzw. Elektrolyt auf.

Dieser Vorgang kann so lange fortgeführt werden, bis das Becherglas mit der Suspension leer ist. Anschließend wird der Pfropfen durch Anlegen des Umgebungsdruckes an den oberen Teil der Messzelle und des durch die Schwerkraft bewirkten Ausströmens des Wassers aus dem Vakuumgefäß entfernt. Der Pfropfen fällt über das Ansaugrohr zurück in das Becherglas. Die Anwendung dieses Funktionsprinzips wird beispielsweise in der DE 102 00 654 A1 beschrieben.

Da in dem Vakuumbehälter der Unterdruck periodisch wechseln muss, um die periodische Flüssigkeitsströmung zu erzeugen, die für die Messung des Strömungspotentials nötig ist, muss das relativ große Volumen des Vakuumbehälters periodisch von einem Unterdruckwert auf den anderen umgeschaltet werden. Um mit der erforderlichen Frequenz arbeiten zu können, ist eine leistungsfähige und große Vakuumpumpe nötig, was besonders nachteilig ist, wenn das Messgerät z. B. durch Chemikalienlieferanten bei verschiedenen Kunden eingesetzt werden muss. Das Gewicht der Vakuumpumpe entspricht etwa dem Gewicht der eigentlichen Messvorrichtung. Das Messgerät wird damit unhandlich und erschwert den Transport der Gesamtausrüstung.

Mit der beschriebenen Ausführungsform nach dem Stand der Technik ist der Unterdruckverlauf an der Messzelle und damit das Strömungspotential periodisch sägezahnförmig ansteigend bzw. abfallend, wobei wegen der begrenzten Leistung der Vakuumpumpe und der erforderlichen Umschaltfrequenz von z. B. 0,5 Hz das Verhältnis von ansteigendem bzw. abfallendem Teil der Druckverlaufs- und Strömungspotential-Kurve zu dem relativ konstanten Teil sehr ungünstig ist bzw. es existiert faktisch kein konstanter Zustand, was eine genaue Berechnung des Zetapotentials sehr erschwert, da wegen des Signalverlaufes die Bearbeitung der Signale zum Beispiel durch Filterung nicht möglich ist. Um trotzdem genaue Messergebnisse zu erhalten, wird beispielsweise die Berechnung des Zetapotentials einschließlich seines Vorzeichens aus dem Strömungspotential- und dem Druckverlauf mittels einer Kreuzkorrelation vorgenommen.

Bei einem weiteren nach der WO 2004/015410 A1 bekannten Verfahren wird der Pfropfen von Hand oder mittels einer Vorrichtung in einem Gefäß erzeugt, das auf der einen Seite mit einem Sieb verschlossen ist und nach Erzeugung des Pfropfens auf der anderen Seite ebenfalls mit einem Sieb abgeschlossen wird. Danach wird Wasser bzw. der Elektrolyt mittels einer oder auch zweier gegenüberliegender motorisch angetriebener Kolbenpumpen mit periodisch wechselnder Richtung durch den Pfropfen gedrückt, wodurch ein periodisches, insbesondere sinusförmiges Strömungspotential entsteht, das mittels Elektroden gemessen wird und aus dem zusammen mit dem gemessenen Differenzdruck das Zetapotential berechnet werden kann.

Bei dieser Ausführungsform nach dem Stand der Technik muss der zu messende Pfropfen von Hand erzeugt werden, da die Messzelle auf beiden Seiten von einem Sieb begrenzt ist. Das ist notwendig, da die Strömungsrichtung des Filtrats / des Elektrolyten in diesem Falle periodisch wechselt.

Es ist Aufgabe der Erfindung, eine gut transportable und kostengünstige Vorrichtung für die Strömungspotentialmessung von Fasern und Partikeln in Suspensionen, insbesondere eine Vorrichtung zur Bestimmung des Zetapotentials der Teilchen von faser- bzw. partikelhaltigen wässrigen Suspensionen durch Messung des Strömungspotentials und einer daraus erfolgenden Berechnung des Zetapotentials mittels einer empirischen Formel, vorzuschlagen. Die Leistungsfähigkeit und Größe der Vakuumpumpe soll aus Portabilitäts- und Kostengründen geringer sein, wobei das Vakuum innerhalb einer Halbperiode gleichbleibend und augenblicklich auf den Sollwert einstellbar sein soll.

Die Lösung dieser Aufgabe erfolgt mit den Merkmalen des Anspruches 1. Vorteilhafte Ausgestaltungen sind in den Unteransprüchen beschrieben. Die erfindungsgemäße Vorrichtung für die Strömungspotentialmessung von Fasern und Partikeln in Suspensionen besteht im Wesentlichen aus mindestens einer Vakuumpumpe, mindestens zwei Vakuumgefäßen mit Drucksensoren, mehreren Ventilsätzen, mindestens einer Druckpumpe, mindestens einer Messzelle mit mindestens einem Sieb und einem in der Messzelle angeordneten Ansaugrohr.

Gemäß der Erfindung werden die Nachteile des Standes der Technik dadurch gelöst, dass ein periodischer Druckverlauf erzeugt wird, indem mindestens zwei Vakuumgefäße mit jeweils verschiedenem konstanten Unterdruck in der Vorrichtung für die Strömungspotentialmessung angeordnet sind. Die Vakuumgefäße werden permanent auf ein jeweils unterschiedliches Vakuum eingestellt und über jeweils ein Ventil im Wechsel an die Messzelle mit dem Pfropfen angeschlossen, wobei die beiden Vakuumgefäße mit Hilfe einer kleinen Vakuumpumpe mit geringer Leistung sowie einer Ventilsteuerung permanent auf dem erforderlichen Unterdruck gehalten werden. Alternativ werden die beiden Vakuumgefäße jeweils mit einer separaten Vakuumpumpe auf dem erforderlichen Unterdruck gehalten. Das durch den Pfropfen strömende Filtrat sammelt sich anteilig in beiden Vakuumgefäßen.

Durch die Umschaltung von einem Gefäß mit definiertem Unterdruck auf das andere erfolgt die Druckänderung am in der Messzelle gebildeten Pfropfen sehr schnell. Der Verlauf des Strömungspotentials und des Differenzdruckes an der Messzelle ist dadurch nicht mehr sägezahnförmig, sondern nahezu rechteckförmig. Dadurch wird die Signalauswertung sehr erleichtert. Die Berechnung mittels Kreuzkorrelation entfällt.

Nach einer weiteren Ausführungsform werden Ventile verwendet, die keine Ein-/Aus-Charakteristik haben, sondern kontinuierlich steuerbar sind und so einen sinusförmigen Druckverlauf an der Messzelle erzeugen können. Auch bei Verwendung derartiger Ventile wird die Signalauswertung sehr erleichtert. Die Berechnung mittels Kreuzkorrelation entfällt.

Die Berechnung des Strömungspotentials kann durch Mittelung über den jeweils konstanten Teil des periodischen Signals und anschließende Berechnung der Differenz erfolgen. Bei einem sinusförmigen Potentialverlauf erfolgt die Berechnung z. B. über den Effektivwert oder den Spitzenwert. Vorteilhaft ist ebenfalls, dass durch die schnelle Anpassung des Druckes an der Messzelle an den Sollwert eine Erhöhung der Messfrequenz erfolgen kann, was zu einer weiteren Verbesserung der Signalauswertung (veränderliche Offsetspannungen und niederfrequente Störungen) führt und auch durch Verringerung der Anzahl der Messperioden zu einer Verkürzung des Messvorganges führt.

Die Portabilität des Gerätes ist erheblich verbessert, da die nunmehr erforderliche kleine Vakuumpumpe auf einfache Weise in das Gerät integriert werden kann. Auch ist die oben beschriebene Alternative realisierbar, indem jeweils eine separate Vakuumpumpe für jeweils ein Vakuumgefäß im Gerät angeordnet wird.

Der Verfahrensablauf für Messungen mit der erfindungsgemäßen Vorrichtung für die Strömungspotentialmessung von Fasern und Partikeln in Suspensionen erfolgt folgendermaßen: Eine oder mehrere in das Messgerät integrierte Vakuumpumpen erzeugen in zwei Vakuumgefäßen, welche an eine Messzelle über Ventile angeschlossen sind, über Drucksensoren und Ventile gesteuert jeweils einen verschiedenen aber konstanten Unterdruck. Durch Zuschalten eines der beiden Vakuumgefäße an die Messzelle wird über ein Ansaugrohr aus einem Becherglas Faserstoffsuspension in diese Messzelle gesaugt. Der obere Teil der Messzelle ist durch ein Sieb abgeschlossen. Dadurch kommt es in der Messzelle zur Bildung eines verdichteten Faserpfropfens.

Durch wechselweise Zuschaltung jeweils eines der zwei Vakuumgefäße, die mit dem oberen Teil der Messzelle über Ventile verbunden sind, kommt es zu einem periodisch wechselnden Unterdruck am oberen Teil der Messzelle und damit zu einer periodischen Strömung von Wasser bzw. Elektrolyt durch den Faserpfropfen. Durch diese Strömung wird ein periodisches Strömungspotential erzeugt, das über zwei jeweils am oberen und unteren Ende der Messzelle befindlichen Elektroden gemessen wird. Aus diesem Strömungspotential und dem Differenzdruck sowie der Leitfähigkeit des Elektrolyten wird mittels einer Formel in einem integrierten Mikrorechner oder einem externen Computer das Zetapotential berechnet.

Nach Abschluss der Messung kann aus einem der beiden Vakuumgefäße mittels eines durch eine Druckpumpe erzeugten Überdruckes Elektrolyt für weitere Messungen entnommen werden.

Zur Entfernung des Faserpfropfens nach Abschluss der Messung und zur Entleerung bzw. Reinigung der Vakuumgefäße, Verbindungsschläuche, Ventile und der Messzelle wird durch die Druckpumpe in den Vakuumgefäßen ein Überdruck erzeugt, der das Filtrat oder eine vorher eingefüllte Reinigungslösung durch die Messanordnung drückt.

Es liegt im Bereich der Erfindung, die Vorrichtung für die Strömungspotentialmessung so zu konzipieren, dass mittels mehrerer Vakuumgefäße für mehrere in Messzellen angeordneter Ansaugrohre Vakuum erzeugt wird und somit eine erhebliche Produktivitätssteigerung erzielt wird. Auch ist erfindungsgemäß vorgesehen, Pumpen zu verwenden, die sowohl als Vakuumpumpe als auch als Druckpumpe arbeiten können. Somit kann die erfindungsgemäße Vorrichtung für bestimmte Einsatzzwecke wiederum leichter gebaut werden.

Es liegt auch weiterhin im Bereich der Erfindung, die Vorrichtung so zu konzipieren, dass mit dieser zusätzlich eine Messung des pH-Wertes erfolgen kann. Dazu ist entweder im unteren Bereich der Messzelle oder am Becherglas mit der Faserstoffsuspension ein Sensor für die Messung des pH-Wertes angeordnet. Eine Anordnung des Sensors zur Messung des pH-Wertes an anderen Stellen mit Zugang zur Faserstoffsuspension oder dem Filtrat ist denkbar. Die Ergebnisse der Messung werden über den Mikrorechner erfasst, von diesem ausgewertet und digital angezeigt.

Um die Vielseitigkeit in der Anwendung der Vorrichtung zu erhöhen, wird wahlweise eine Dosiervorrichtung in Verbindung mit einer Rührvorrichtung, beispielsweise einem Magnetrührer, im erfindungsgemäßen Gerät integriert. Mit dieser Dosiervorrichtung können der Faserstoffsuspension Additive zugeführt werden. Es handelt sich dabei um Additive, die im realen Prozess der Papierherstellung zusätzlich der Faserstoffsuspension zu gegeben werden, um dem herzustellenden Papier die gewünschte Konsistenz, Struktur und chemische Zusammensetzung zu geben. Die Dosiervorrichtung in Verbindung mit der Rührvorrichtung wird an oder unmittelbar neben der Messzelle angeordnet, so dass die zuzugebenden Additive direkt in das Becherglas gelangen. Es ist auch denkbar, die Dosiervorrichtung mit der Rührvorrichtung flexibel am Becherglas zu arretieren.

Die Erfindung wird im Folgenden anhand des in der Zeichnung dargestellten Ausführungsbeispieles näher erläutert und beschrieben. Die der Zeichnung und der Beschreibung zu entnehmenden Merkmale können bei anderen Ausführungsformen der Erfindung einzeln für sich oder zu mehreren in beliebigen Kombinationen Anwendung finden.

Die Zeichnung zeigt in
- Figur 1: eine schematische Darstellung einer Vorrichtung für die Strömungspotentialmessung von Fasern und Partikeln in Suspensionen mit einer Vakuumpumpe und
- Figur 2: eine schematische Darstellung einer Vorrichtung für die Strömungspotentialmessung von Fasern und Partikeln in Suspensionen mit zwei Vakuumpumpen.

### Beispiel 1

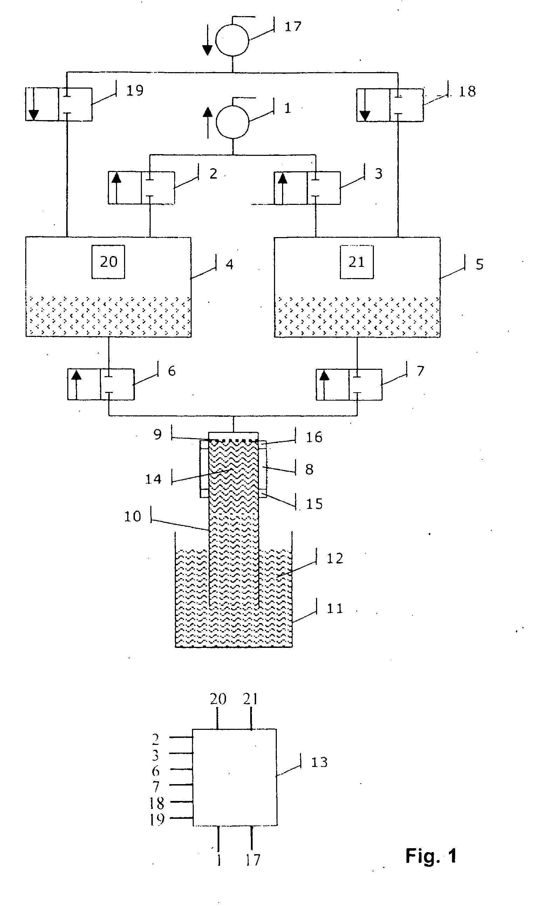

Die erfindungsgemäße Vorrichtung für die Strömungspotentialmessung von Fasern und Partikeln in Suspensionen wird nachfolgend anhand der Figur 1 näher erläutert. Sie besteht im Wesentlichen aus einer Vakuumpumpe 1, zwei Vakuumgefäßen 4; 5 mit Drucksensoren 20; 21, mehreren Ventilsätzen, einer Druckpumpe 17, einer Messzelle 8 mit einem Sieb 9 und einem in der Messzelle 8 angeordneten Ansaugrohr 10.

Die Vakuumpumpe 1 wird über die Ventile 2 und 3 an zwei Vakuumgefäße 4 und 5 angeschlossen, in denen gesteuert über Drucksensoren 20 und 21 und die Ventile 2 und 3 jeweils ein konstantes Vakuum erzeugt wird. Diese Vakuumgefäße 4 und 5 wiederum werden über die Ventile 6 und 7 an die Messzelle 8 angeschlossen, deren Oberseite mit einem Sieb 9 abgeschlossen ist, und an der sich das Ansaugrohr 10 befindet, das in das Becherglas 11 mit der zu messenden Suspension 12 ragt. Alle Ventile werden über Mikrorechner 13 so gesteuert, dass am zuerst durch permanentes Ansaugen von Suspension gebildeten Faserpfropfen 14 ein periodisches Strömungspotential über zwei aus beispielsweise Edelstahl bestehenden Elektroden 15 und 16 gemessen werden kann. Aus dem Strömungspotential wird zusammen mit weiteren gemessenen Parametern, z. B. Verlauf des Differenzdruckes und der Leitfähigkeit des Elektrolyten, ein Zetapotential berechnet.

Die Entfernung des Pfropfens 14 aus der Messzelle 8 erfolgt über eine kleine Druckpumpe 17, die über zwei Ventile 18 und 19 an die beiden Vakuumgefäße 4 und 5 angeschlossen ist. Die Druckpumpe 17 erzeugt einen geringen Überdruck, der das Filtrat aus den beiden Vakuumgefäßen 4 und 5 entfernt und damit zusammen auch den Pfropfen 14 aus der Messzelle 8 drückt. Die Druckpumpe 17 kann auch dazu dienen, Filtrat für weitere Messungen mit anderen Messgeräten aus einem der Vakuumgefäße 4 bzw. 5 zu entnehmen.

Die Messung mit der erfindungsgemäßen Vorrichtung für die Strömungspotentialmessung von Fasern und Partikeln in Suspensionen 12 wird durch die im Messgerät integrierte Vakuumpumpe 1 dadurch ermöglicht, dass die Vakuumpumpe 1 in zwei Vakuumgefäßen 4; 5, welche an eine Messzelle 8 über Ventile 6; 7 angeschlossen sind, über Drucksensoren 20; 21 und Ventile 2; 3 gesteuert, jeweils einen verschieden konstanten Unterdruck erzeugt. Durch Zuschalten eines der beiden Vakuumgefäße 4; 5 an die Messzelle 8 wird über ein Ansaugrohr 10 aus einem Becherglas 11 Faserstoffsuspension 12 in die Messzelle 8 gesaugt. Der obere Teil der Messzelle 8 ist durch ein Sieb 9 abgeschlossen. Dadurch kommt es in der Messzelle 8 zur Bildung eines verdichteten Faserpfropfens 14.

Durch wechselweise Zuschaltung jeweils eines der zwei Vakuumgefäße 4; 5, die mit dem oberen Teil der Messzelle 8 über Ventile 6; 7 verbunden sind, kommt es zu einem periodisch wechselnden Unterdruck am oberen Teil der Messzelle 8 und damit zu einer periodischen Strömung von Wasser bzw. Elektrolyt durch den Faserpfropfen 14. Durch diese Strömung wird ein definiertes Strömungspotential erzeugt, das über zwei jeweils am oberen und unteren Ende der Messzelle befindlichen Elektroden 15; 16 gemessen wird. Aus diesem Strömungspotential und dem Differenzdruck sowie der Leitfähigkeit des Elektrolyten wird mittels einer Formel in einem integrierten Mikrorechner 13 das Zetapotential berechnet.

Zur Entfernung des Faserpfropfens 14 wird nach Abschluss der Messung und zur Entleerung bzw. Reinigung der Vakuumgefäße 4; 5 der Verbindungsschläuche, Ventilsätze und der Messzelle 8 durch die Druckpumpe 17 in den Vakuumgefäßen 4; 5 ein Überdruck erzeugt, der das Filtrat oder eine vorher eingefüllte Reinigungslösung durch die Messanordnung drückt. Nach Abschluss der Messung kann aus einem der beiden Vakuumgefäße 4; 5 mittels eines durch die Druckpumpe 17 erzeugten Überdruckes Elektrolyt für weitere Messungen entnommen werden.

### Beispiel 2

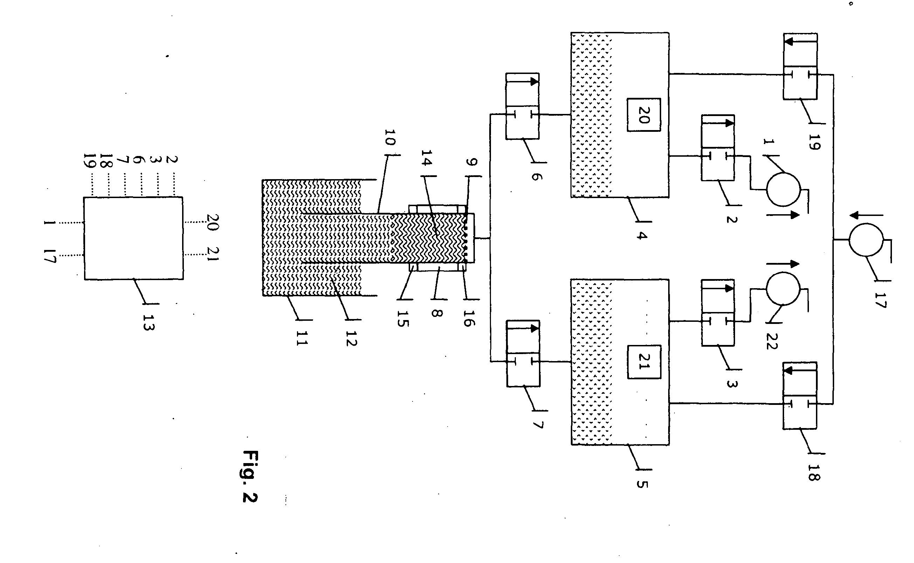

In diesem Ausführungsbeispiel wird das Messgerät mit mindestens zwei Vakuumpumpen 1; 22 nach Figur 2 näher erläutert. Die erfindungsgemäße Vorrichtung für die Strömungspotentialmessung von Fasern und Partikeln in Suspensionen nach diesem Ausführungsbeispiel besteht im Wesentliche aus zwei Vakuumpumpen 1 und 22, zwei Vakuumgefäßen 4; 5 mit Drucksensoren 20; 21, mehreren Ventilsätzen, einer Druckpumpe 17, einer Messzelle 8 mit einem Sieb 9 und einem in der Messzelle 8 angeordneten Ansaugrohr 10.

Die Vakuumpumpen 1; 22 werden über die Ventile 2 und 3 an zwei Vakuumgefäße 4 und 5 angeschlossen, in denen gesteuert über Drucksensoren 20 und 21 und die Ventile 2 und 3 jeweils ein konstantes Vakuum erzeugt wird. Diese Vakuumgefäße 4 und 5 wiederum werden über die Ventile 6 und 7 an die Messzelle 8 angeschlossen, deren Oberseite mit einem Sieb 9 abgeschlossen ist, und an der sich das Ansaugrohr 10 befindet, das in das Becherglas 11 mit der zu messenden Suspension 12 ragt. Alle Ventile werden über Mikrorechner 13 so gesteuert, dass am zuerst durch permanentes Ansaugen von Suspension gebildeten Faserpfropfen 14 ein periodisches Strömungspotential über zwei aus beispielsweise Edelstahl bestehenden Elektroden 15 und 16 gemessen werden kann. Aus dem Strömungspotential wird zusammen mit weiteren gemessenen Parametern, z. B. Verlauf des Differenzdruckes und der Leitfähigkeit des Elektrolyten, ein Zetapotential berechnet.

Die Messung mit der erfindungsgemäßen Vorrichtung für die Strömungspotentialmessung von Fasern und Partikeln in Suspensionen 12 nach diesem Ausführungsbeispiel wird durch die im Messgerät integrierten Vakuumpumpen 1 und 22 dadurch ermöglicht, dass die Vakuumpumpen 1 und 22 in zwei Vakuumgefäßen 4; 5, welche an eine Messzelle 8 über Ventile 6; 7 angeschlossen sind, über Drucksensoren 20; 21 und Ventile 2; 3 gesteuert, jeweils einen verschiedenen aber konstanten Unterdruck erzeugen. Der weitere Verfahrensablauf und die Entfernung des Pfropfens 14 aus der Messzelle 8 erfolgt wie in Beispiel 1 beschrieben.

## Patentansprüche

1. Vorrichtung für die Strömungspotentialmessung von Fasern und Partikeln in Suspensionen, enthaltend eine Messzelle (8) mit Sieb (9) und Elektroden (15, 16) und ein Ansaugrohr (10), **dadurch gekennzeichnet dass** eine in der Vorrichtung integrierte Vakuumpumpe (1) über wenigstens jeweils ein Ventil (2, 3) enthaltende Leitungen mit mindestens zwei Vakuumgefäßen (4; 5) verbunden ist, und jedes der Vakuumgefäße (4; 5) jeweils über Leitungen mit zwischengeschalteten Ventilen (6: 7) mit der Messzelle (8) in Verbindung steht, wobei während eines Messvorganges in dem Vakuumgefäß (4) ein definiert einstellbarer und in dem Vakuumgefäß (5) ebenfalls ein definiert einstellbarer aber gegenüber dem Vakuumgefäß (4) unterschiedlicher Unterdruck besteht.

2. Vorrichtung für die Strömungspotentialmessung von Fasern und Partikeln in Suspensionen, enthaltend eine Messzelle (8) mit Sieb (9) und Elektroden (15, 16) und ein Ansaugrohr (10), **dadurch gekennzeichnet dass** mindestens zwei in der Vorrichtung integrierte Vakuumpumpen (1; H 22) separat jeweils mit mindestens einem Vakuumgefäß (4; 5) über Leitungen verbunden sind, und jedes der Vakuumgefäße (4; 5) jeweils über Leitungen mit zwischengeschalteten Ventilen (6; 7) mit der Messzelle (8) in Verbindung steht, wobei während eines Messvorganges in dem Vakuumgefäß (4) ein definiert einstellbarer und in dem Vakuumgefäß (5) ebenfalls ein definiert einstellbarer aber gegenüber dem Vakuumgefäß (4) unterschiedlicher Unterdruck besteht.

3. Vorrichtung für die Strömungspotentialmessung nach Anspruch 1 oder 2, wobei die Vakuumgefäße (4; 5) über Leitungen mit zwischengeschalteten Ventilen (18; 19) mit einer Druckpumpe (17) in Verbindung stehen.

4. Vorrichtung nach Anspruch 1 oder 2, wobei jedes der Vakuumgefäße (4; 5) über Leitungen mit zwischengeschalteten Ventilen (18; 19) mit einer separat angeordneten Druckpumpe (17) in Verbindung steht.

5. Vorrichtung nach Anspruch 1 oder 2, wobei die Vakuumgefäße (4; 5) mit Drucksensoren (20; 21) in Verbindung stehen.

6. Vorrichtung nach einem oder mehreren der vorgenannten Ansprüche 1 bis 5, wobei eine Pumpvorrichtung angeordnet ist, die wahlweise der Vakuumerzeugung oder Druckerzeugung dient.

7. Vorrichtung nach einem oder mehreren der vorgenannten Ansprüche 1 bis 6, wobei zwischen den Vakuumpumpen (1; 22) und dem Vakuumgefäß (4; 5) jeweils Ventile (2; 3) angeordnet sind.

8. Vorrichtung nach einem oder mehreren der vorgenannten Ansprüche 1 bis 7, wobei in der Vorrichtung mindestens zwei Messzellen (8) mit separaten Ansaugrohren (10) angeordnet sind.

9. Vorrichtung nach einem oder mehreren der vorgenannten Ansprüche 1 bis 8, wobei in der Vorrichtung oder in Kombination mit dieser, ein Sensor für die Messung des pH-Wertes angeordnet ist und die Messergebnisse von einem Mikrorechner (13) erfasst, von diesem ausgewertet und digital angezeigt werden.

10. Vorrichtung nach einem oder mehreren der vorgenannten Ansprüche 1 bis 9, wobei in der Vorrichtung oder in Kombination mit dieser eine Dosiervorrichtung, vorzugsweise für die Zugabe von Additiven in Verbindung mit einer Rührvorrichtung angeordnet ist.

## Claims

1. A device for measuring the streaming potential of fibres and particles in suspensions, containing a measuring cell (8) including a sieve (9) and electrodes (15, 16), and a suction pipe (10), **characterized in that** a vacuum pump (1) which is integrated in the device is connected to at least two vacuum vessels (4; 5) via pipes each of which contains at feast one valve (2, 3), and each of the vacuum vessels (4; 5) is connected to the measuring cell (8) via pipes having valves (6; 7) installed therein in each case, wherein during a measuring process there is a negative pressure which can be adjusted in a defined manner in the vacuum vessel (4) and another negative pressure in the vacuum vessel (5) which can also be adjusted in a defined manner, but which is different from that in the vacuum vessel (4).

2. A device for measuring the streaming potential of fibres and particles in suspensions, containing a measuring cell (8) including a sieve (9) and electrodes (15, 16), and a suction pipe (10), **characterized in that** at least two vacuum pumps (1; 22) which are integrated in the device are connected separately to at least one vacuum vessel (4; 5) in each case via pipes, and each of the vacuum vessels (4; 5) is connected to the measuring cell (8) via pipes having valves (6; 7) installed therein in each case, wherein during a measuring process there is a negative pressure which can be adjusted in a defined manner in the vacuum vessel (4) and another negative pressure in the vacuum vessel (5) which can also be adjusted in a defined manner, but which is different from that in the vacuum vessel (4).

3. A device for measuring the streaming potential according to claim 1 or 2, wherein the vacuum vessels (4; 5) are connected to a pressure pump (17) via pipes having valves (18; 19) installed therein.

4. A device according to claim 1 or 2, wherein each of the vacuum vessels (4; 5) is connected to a separate pressure pump (17) via pipes having valves (18; 19) installed therein.

5. A device according to claim 1 or 2, wherein the vacuum vessels (4; 5) are connected to pressure sensors (20; 21).

6. A device according to any one or several of the preceding claims 1 to 5, wherein a pumping device is arranged which selectively serves to generate a vacuum or to generate pressure.

7. A device according to any one or several of the preceding claims 1 to 6, wherein valves (2; 3) are arranged in each case between the vacuum pumps (1; 22) and the vacuum vessel (4; 5).

8. A device according to any one or several of the preceding claims 1 to 7, wherein at least two measuring cells (8) having separate suction pipes (10) are arranged in the device.

9. A device according to any one or several of the preceding claims 1 to 8, wherein a sensor for measuring the pH value is arranged in the device or in combination therewith, and the measuring results are detected, evaluated, and displayed digitally by a micro-computer (13).

10. A device according to any one or several of the preceding claims 1 to 9, wherein a dosing device, which preferably serves to add additives and cooperates with a stirring device, is arranged in the device or in combination therewith.

## Revendications

1. Dispositif pour la mesure de potentiel d'écoulement de fibres et de particules en suspension, comprenant une cellule de mesure (8) avec tamis (9) et électrodes (15, 16) et un tube d'aspiration (10), **caractérisé en ce qu'**une pompe à vide (1) intégrée au dispositif est raccordée par des conduites contenant au moins respectivement une vanne (2, 3) avec au moins deux cuves à vide (4; 5), et **en ce que** chacune des cuves à vide (4; 5) est respectivement raccordée à la cellule de mesure (8) par des conduites avec vannes (6; 7) intermédiaires, une sous-pression réglable de façon définie étant présente dans la cuve à vide (4) et également une sous-pression réglable de façon définie mais différente par rapport à la cuve à vide (4) étant présente dans la cuve à vide (5), durant un processus de mesure.

2. Dispositif pour la mesure de potentiel d'écoulement de fibres et de particules en suspension, comprenant une cellule de mesure (8) avec tamis (9) et électrodes (15, 16) et un tube d'aspiration (10), **caractérisé en ce qu'**au moins deux pompes à vide (1 ; 22) intégrées au dispositif sont raccordées séparément par des conduites avec au moins une cuve à vide (4; 5), et **en ce que** chacune des cuves à vide (4; 5) est respectivement raccordée à la cellule de mesure (8) par des conduites avec vannes (6; 7) intermédiaires, une sous-pression réglable de façon définie étant présente dans la cuve à vide (4) et également une sous-pression réglable de façon définie mais différente par rapport à la cuve à vide (4) étant présente dans la cuve à vide (5), durant un processus de mesure.

3. Dispositif pour la mesure de potentiel d'écoulement selon la revendication 1 ou 2, les cuves à vide (4; 5) étant connectées à une pompe de refoulement (17) par des conduites avec vannes intermédiaires (18; 19).

4. Dispositif selon la revendication 1 ou 2, chacune des cuves à vide (4; 5) étant connectée à une pompe de refoulement (17) disposée séparément par des conduites avec vannes intermédiaires (18; 19).

5. Dispositif selon la revendication 1 ou 2, les cuves à vide (4; 5) étant connectées à des capteurs de pression (20; 21).

6. Dispositif selon l'une ou plusieurs des revendications précédentes 1 à 5, un dispositif de pompage étant disposé pour servir à la génération de vide ou à la génération de pression, au choix.

7. Dispositif selon l'une ou plusieurs des revendications précédentes 1 à 6, des vannes (2; 3) étant respectivement disposées entre les pompes à vide (1; 22) et la cuve à vide (4; 5).

8. Dispositif selon l'une ou plusieurs des revendications précédentes 1 à 7, au moins deux cellules de mesure (8) avec tubes d'aspiration (10) séparés étant disposées dans le dispositif.

9. Dispositif selon l'une ou plusieurs des revendications précédentes 1 à 8, un capteur pour la mesure du pH étant disposé dans le dispositif ou en combinaison avec celui-ci, et les résultats de mesure étant saisis, évalués et affichés de façon numérique par un microcalculateur (13).

10. Dispositif selon l'une ou plusieurs des revendications précédentes 1 à 9, un dispositif de dosage étant disposé dans le dispositif ou en combinaison avec celui-ci, de préférence pour l'ajout d'additifs en connexion avec un dispositif d'agitation.
